# EUROPÄISCHE PATENTSCHRIFT

(11) **EP 1 114 186 B1**
(45) Veröffentlichungstag und Bekanntmachung des Hinweises auf die Patenterteilung: **05.07.2006**
(21) Anmeldenummer: 99955728.3
(22) Anmeldetag: 15.09.1999
(51) Int. Cl.: C12Q 1/68, B01J 19/00

(54) **VERFAHREN ZUR QUALITÄTSKONTROLLE BEIM AUFBAU VON OLIGOMERRASTERN**
METHOD FOR CONTROLLING QUALITY IN THE CONSTRUCTION OF OLIGOMER GRIDS
PROCEDE DE CONTROLE QUALITE LORS DE LA CONSTITUTION DE TRAMES OLIGOMERIQUES

(30) Priorität: 15.09.1998 DE 19842164
(43) Veröffentlichungstag der Anmeldung: 11.07.2001
(73) Patentinhaber: Deutsches Krebsforschungszentrum Stiftung des öffentlichen Rechts, 69120 Heidelberg (DE)
(72) Erfinder: BEIER, Markus, D-69120 Heidelberg (DE)
(74) Vertreter: Schüssler, Andrea
(86) Internationale Anmeldenummer: PCT/DE1999/002975
(87) Internationale Veröffentlichungsnummer: WO 2000/015837

(56) Entgegenhaltungen:
- EP-A- 0 475 443
- EP-A- 0 818 460
- WO-A-94/08047
- WO-A-97/39151
- DE-A- 19 625 397
- WEILER J ET AL: "COMBINING THE PREPARATION OF OLIGONUCLEOTIDE ARRAYS AND SYNTHESIS OF HIGH-QUALITY PRIMERS" ANALYTICAL BIOCHEMISTRY,US,ACADEMIC PRESS, SAN DIEGO, CA, Bd. 243, Nr. 2, 15. Dezember 1996 (1996-12-15), Seiten 218-227, XP000684351 ISSN: 0003-2697
- WEILER J UND HOHEISEL J D: "Picomole synthesis of high quality oligonucleotide primers in combination with the preparation of oligonucleotide arrays" NUCLEOSIDES & NUCLEOTIDES, Bd. 16, Nr. 7-9, 1997, Seiten 1793-1796, XP002132646

## Beschreibung

Die Erfindung betrifft ein Verfahren zur Qualitätskontrolle beim Aufbau von Oligomerrastern.

Für diagnostische Screenings werden Mikrochips mit Oligomeren in Form von Rastern beschichtet (Oligomerchips/Biochips). Mit diesen kann dann eine Probe nach einem passenden, d.h. damit hybridisierenden, Molekül abgesucht werden. Solche Oligomerraster auf einem Chip können aus Nucleinsäureoligonukleotiden, wie DNA-, RNA- oder Nucleinsäurebiopolymeren oder analogen Verbindungen dazu bestehen, die auf einer festen Phase aufgebracht sind.

Die Fixierung der Oligomeren erfolgt nicht immer quantitativ, so daß nicht immer Chips mit dem gleichen Beschichtungsgrad erhalten werden. Nach dem Aufbau solcher Oligomerraster muß daher überprüft werden, ob die Synthese erfolgreich verlaufen ist, bzw. der Grad der erfolgreichen Synthese muß ermittelt werden. Bisher wurden hierzu mit einem permanenten (Fluoreszenz)label versehene Phosphatreagenzien verwendet. Da diese Label nicht wieder abgespalten werden konnten, beeinträchtigen sie ggf. die anschließende Verwendung des Biochips. Bekannt ist auch die Machierung von Oligonukleotiden mit mehreren Biotinen wobei eine spezielle Anzahl von Bahen zwischen zwei Biotinen vorliegen sollte (JP 6100 586 A).

Ein definierter Qualitätsassay für den Aufbau von Oligomerrastern auf einer Chipoberfläche, der keine störenden Nebeneffekte auslöst, ist daher bisher nicht bekannt. Auf dem noch in der Entwicklung begriffenen Gebiet der Biochip-Technologie ist jedoch eine Qualitätskontrolle unumgänglich, um eine reproduzierbare Herstellung gleicher Qualität zu gewährleisten.

Der Erfindung liegt daher die Aufgabe zugrunde, ein Verfahren zur Qualitätskontrolle von Oligomerrastern bereitzustellen. Das Verfahren soll universell einsetzbar sein und sich mit geringem Aufwand rasch durchführen lassen. Ferner soll durch die Qualitätskontrolle die anschließende Verwendung als Biochip nicht beeinträchtigt werden. Das Verfahren soll sich für alle Arten von Oligomerrastern eignen.

Diese Aufgabe wird durch ein Verfahren gemäß Patentanspruch 1 gelöst. Vorteilhafte Ausgestaltungen ergeben sich aus den Unteransprüchen.

Überraschenderweise wurde gefunden, daß eine Qualitätskontrolle ohne Beeinträchtigung der weiteren Verwendung des Chips durchgeführt werden kann, wenn an das sich auf dem Chip befindliche Oligomerraster eine Phosphateinheit ankondensiert wird, die reversibel mit einer signalgebenden Reportergruppe versehen ist bzw. mit einer solchen verbunden wird. Durch Detektion des Signals der an die Oligomeren an den Rasterpositionen gebundenen Reportergruppe kann der Erfolg der Synthese kontrolliert werden, bzw. verschiedene Rasterpositionen können miteinander verglichen werden. Nach erfolgter Qualitätskontrolle wird die Reportergruppe wieder abgelöst.

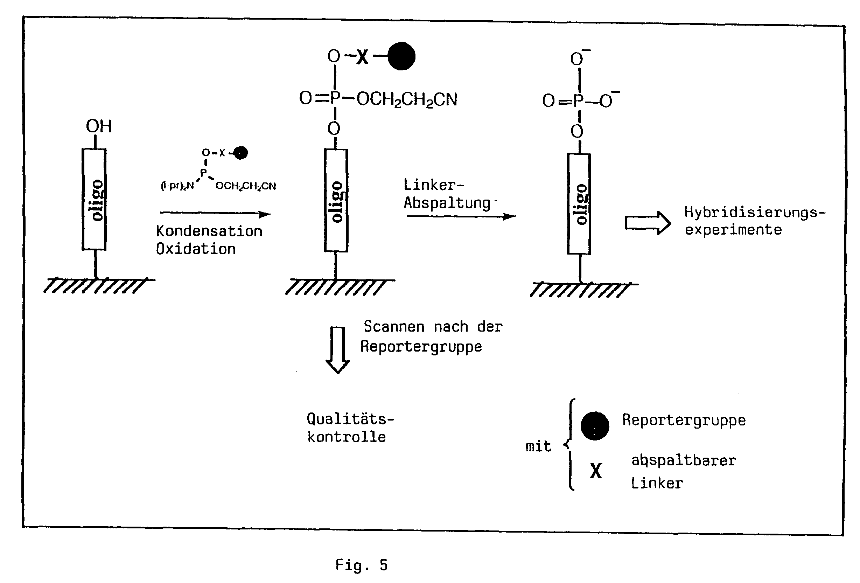

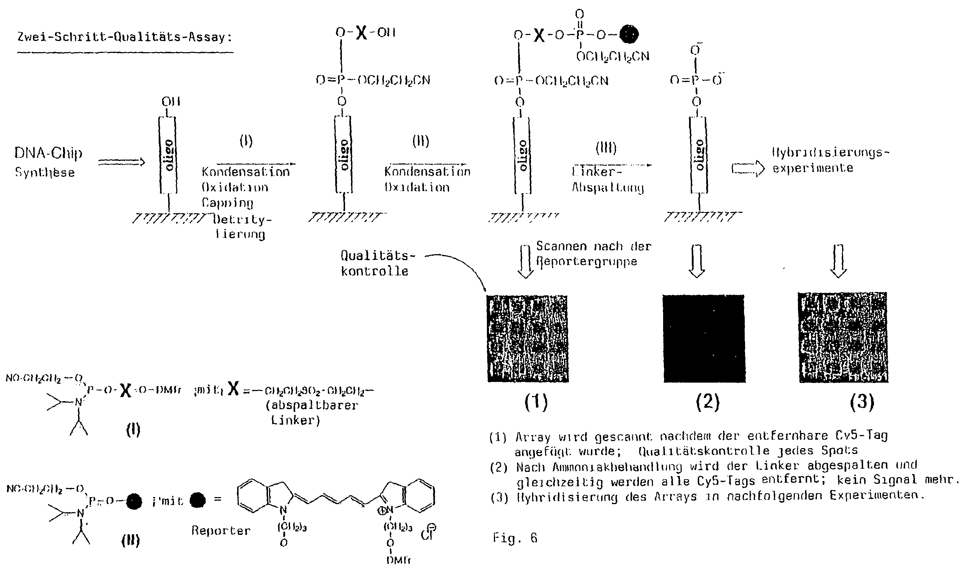

Die Erfindung betrifft somit ein Verfahren zur Qualitätskontrolle von Oligomerrastern, das dadurch gekennzeichnet ist, daß man an Oligonukleotide an bestimmten Rasterpositionen (vorzugsweise an alle oder an eine vorher ausgewählte Anzahl) eine Phosphateinheit, die mit einer signalgebenden Reportergruppe verknüpft ist bzw. wird, ankondensiert, anhand des Signals der Reportergruppe den Grad der Oligomersynthese bestimmt und anschließend die Reportergruppe wieder abspaltet. Ein allgemeines Schema ist in Fig. 5 bzw. 6 gezeigt.

Durch Detektion der von den besetzten Rasterpositionen ausgehenden Signalen der Reportergruppe kann der Umfang des Syntheseerfolgs kontrolliert werden. Nach erfolgter Qualitätskontrolle kann die Reportergruppe wieder abgespalten werden und führt so bei den anschließenden Experimenten zu keinerlei störenden Effekten. Lediglich die Phosphatgruppe bleibt mit den Oligomeren verbunden. Sie stört jedoch die nachfolgende Verwendung, z.B. bei einer Hybridisierung, nicht, sondern erhöht ggf. vorteilhafterweise zudem noch die Schmelztemperatur des an die feste Phase gebundenen Oligomers.

Erfindungsgemäß soll unter einer Phosphateinheit eine solche durch Methoden der Phosphoramidit-Chemie aufkondensierte Einheit verstanden werden. Desweiteren können die Phosphateinheiten auch durch bekannte Verfahren der Phosphormonoester-, Phosphordiester- oder H-Phosphonat-Chemie erzeugt werden.

Nachfolgend wird die Erfindung anhand von Phosphoramidit als Basis der Phosphatgruppe beschrieben, was erfindungsgemäße bevorzugt ist. Dies ist jedoch nicht als Beschränkung auszulegen. Erfindungsgemäß wird die Phosphateinheit durch Reaktion des bereits an der festen Phase befindlichen Oligomers an dessen 5'-oder 3'-Ende mit dem Phosphoramidit nach Oxidation erzeugt. Hierbei wird das Phosphoramidit unter Zuhilfenahme eines aciden Katalysators (z.B. Tetrazol, Tetrazol-Derivate, Pyridinhydrochlorid) mit dem an der festen Phase befindlichen Oligomer-Strang umgesetzt. Nachfolgende Oxidation, z.B. mit Jod, tert-Butylhydroperoxid etc., ergibt eine stabile Phosphor(V)-Verbindung (=Phosphateinheit). Vorteilhaft erweist sich, daß man sich hierbei der üblichen Schritte der Synthese von Oligonukleotiden bedienen kann, die sich problemlos auf einem kommerziell erhältlichen DNA/RNA-Synthesizer automatisieren lassen. Hierbei sind keine Abänderungen der üblicherweise verwendeten Reagenzien oder Syntheseprotokolle nötig.

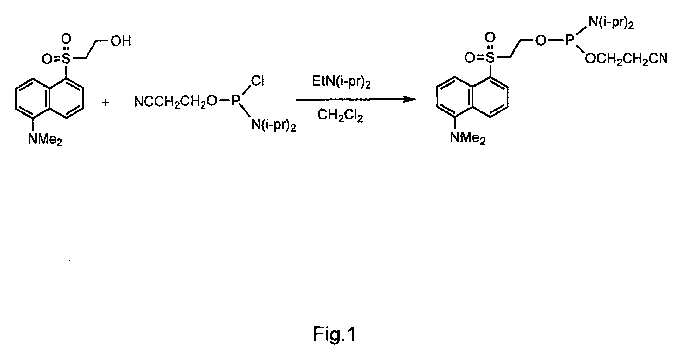

Die signalgebende Reportergruppe kann jedes beliebige signalgebende Molekül sein, das sich über einen entsprechenden Linker an ein Phosphoramidit ankoppeln läßt. Der Linker sorgt dafür, daß nach erfolgter Umsetzung des Phosphoramidits mit dem Oligomer auf der festen Phase und nach Detektion der Reportergruppe eine Abspaltung der Reportergruppe erfolgen kann. Signalgebende Reportergruppen können beliebige fluoreszierende, farbgebende, radioaktive, chemolumineszierende Verbindungen sein. Bevorzugt sind fluoreszierende Verbindungen, wie Dansylethanol, Fluorescein oder Pyren. Beispiele für weitere Reportergruppen sind für die Ankopplung an die Phosphatgruppe geeignet derivatisierte Derivate von Cy3, Cy5, Dabsylchlorid, TAMRA, Hexachlorofluorescein. Erfindunggemäß kann die Reportergruppe bereits mit der Phosphateinheit verbunden sein, wenn diese mit dem Oligomer verbunden wird (1-stufiger Prozeß, s. Fig. 1 und 5) oder es kann der Linker mittels einer Phosphateinheit an das Oligomer gebunden werden bevor die Reportergruppe mittels einer weiteren Phosphateinheit angeknüpft wird (2-stufiger Prozeß, s. Fig. 6).

Der 1-stufige Prozeß ist ausführlich in Beispiel 1 beschrieben. Kennzeichnend für den 1-stufigen Prozeß ist, daß das Reportermolekül (z.B. Fluoreszenz-Tag) und der abspaltbare Linker Teile eines einzigen chemischen Moleküls (z.B. 2-Cyanoethyl-2-dansylethyl-N,N-diisopropyl-phosphoramidit) sind. Dieses wird im Rahmen des letzten Schrittes der Oligonukleotid-Synthese auf den Chip kondensiert. Nach Oxidation, um einen stabilen Phosphodiester zu bilden, wird der Chip mittels Nachweis der Reportergruppe auf seine Qualität gecheckt. Danach wird die Reportergruppe, z.B. durch Basenbehandlung, entfernt. Vorzugsweise, aber nicht notwendigerweise, können die Phosphatschutzgruppen im gleichen Schritt oder erst später entfernt werden. Zurückgelassen wird eine Phosphateinheit als Anhängsel an einem Ende des jeweiligen Oligomerstrangs. Dann kann der Array in Standardexperimenten (z.B. in Hybridisierungen) eingesetzt werden.
Beim 1-stufigen Prozeß enthält der Linker eine durch Säure, Base oder Licht spaltbare Einheit (nachstehend: X), z.B. Sulfonylethyl, 2-(2,2-dicarboxyethyl)propyl, 2-(2-nitrophenyl)propyl, 2-(2-nitrophenyl)ethyl. Desweiteren enthält der Linker für die Anknüpfung der signalgebenden Reportergruppe eine Hydroxy- oder Amino-Funktion (nachstehend: H). Weiter enthält der Linker einen Spacer (nachstehend: M), der die spaltbare Einheit (X) von der Amino- oder Hydroxylfunktion (H) räumlich trennt. Der Linker (L) kann deshalb durch folgende allgemeine Formel wiedergegeben werden:

L = H-M-X-

mit
X = spaltbare Einheit
H = Hydroxyl oder Amino
M = Alkyl-, Aryl, usw.

Vor der Kondensation an den Oligomerarray wird ein Reagenz gebildet, bei dem der Linker mit einer Phosphitamid-Einheit und mit der Reportergruppe verbunden ist. Die Anknüpfung der Reportergruppe erfolgt mittels geeigneter Reporter-Derivate. Bevorzugt sind Anknüpfungen an die Hydroxy- oder Amino-Funktion (H) über eine Ester- oder Amid- oder Sulfonamid-Brücke, da hierfür kommerziell geeignete derivatisierte Verbindungen (z.B. Carbonsäuren, Sulfonylchloride) nahezu aller bekannten Reportergruppen verfügbar sind. Das Reagenz hat folgende allgemeine Formel: 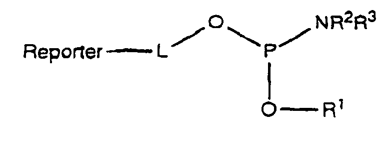
L = spaltbare Linker-Einheit
R¹ = Phosphatschutzgruppe, z.B. β-Cyanoethyl, 2-(4-Nitrophenyl)ethyl, 2-(4-cyanophenyl)ethyl
R² = Isopropyl-, Ethyl, Methyl-
R³ = Isopropyl-, Ethyl, Methyl-
Reporter = signalgebende Reportereinheit

Beim 1-stufigen Prozeß ist ein Linkersystem vom Typ 2-(4-Aminophenylsulfonyl)ethyl- bevorzugt, wobei auch andere dem Fachmann bekannte spaltbare Linker einsetzbar sind. Die Verwendung von 2-(4Aminophenylsulfonyl)ethanol erlaubt durch Umsetzung der Aminofunktion mit signalgebenden Reportergruppen, die eine Sulfonyl- oder Carboxylgruppe tragen, den Zugang zu nahezu allen bekannten signalgebenden Reportergruppen.

Beim 2-stufigen Prozeß (s. Fig. 6) sind das Reportermolekül (z.B. Fluoreszenz-Tag) und der abspaltbare Linker (z.B. Sulfonylethyl-Linker) Teile von zwei verschiedenen chemischen Molekülen.
Als Linker im 2-stufigen Prozeß kommen bevorzugt Phosphateinheiten mit spaltbaren Einheiten zum Einsatz. Die Linker (nachstehend: L) haben bevorzugt für die Anknüpfung der nächsten Phosphateinheit eine geschützte Hydroxyl- oder Aminofunktion (nachstehend: H). Geeignete Hydroxyl- oder Amino-Schutzgruppen (nachstehend: G) sind dem Fachmann bekannt und sind z.B. säurelabil (z.B. Dimethoxytrityl, Monomethoxytrityl), basenlabil (z.B. Fmoc) oder photolabil (z.B. MeNPOC, NPPOC). Der Linker enthält weiter eine durch Säure, Base oder Licht spaltbare Einheit (nachstehend: X), z.B. Sulfonylethyl, 2-(2,2-dicarboxyethyl)propyl, 2-(2-nitrophenyl)propyl, 2-(2-nitrophenyl)ethyl). Weiter enthält der Linker einen Spacer (nachstehend: M), der die spaltbare Einheit (X) von der Amino- oder Hydroxylfunktion (H) räumlich trennt. Der Linker (L) kann deshalb durch folgende allgemeine Formel wiedergegeben werden:

L = G-H-M-X-

mit
X = spaltbare Einheit
H = Hydroxyl oder Amino
G = Schutzgruppe für H
M = Alkyl-, Aryl, usw.

Der Linker wird mit einer Phosphitamid-Einheit verbunden. Das entstehende Linker-Phosphoramidit-Reagenz wird dann wie vorstehend beschrieben an den OligomerArray kondensiert. Das Linker-Phosphoramidit-Reagenz hat vorzugsweise folgende allgemeine Formel: 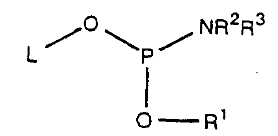
L = geschützter spaltbarer Linker (s.oben)
R¹ = Phosphatschutzgruppe, z.B. β-Cyanoethyl, 2-(4-Nitrophenyl)ethyl, 2-(4-cyanophenyl)ethyl
R² = Isopropyl-, Ethyl, Methyl-
R³ = Isopropyl-, Ethyl, Methyl-

Bevorzugte Linker-Phosphoramidit-Verbindungen im 2-stufigen Prozeß sind:
2-[2-(4,4'-dimethoxytrityloxy)ethylsulfonyl]ethyl-(2-cyanoethyl)-(N,N-diisopropyl)-phosphoramidit (Eurogentec, Liege. Belgium) 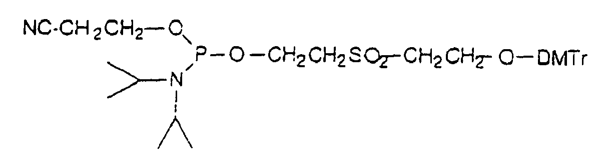
[3-(4,4'-Dimethoxytrityloxy)-2,2-dicarboxyethyl]propyl-(2-cyanoethyl)-N,N-diisopropyl)phosphoramidite

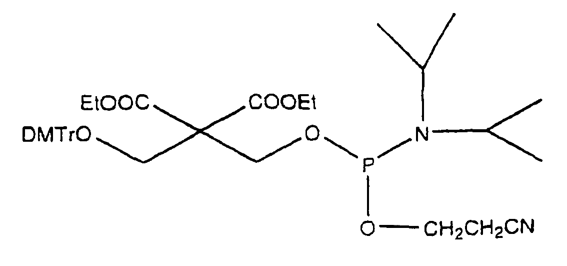

Eine bevorzugte Ausführungsform des 2-stufigen Prozeßes ist in Fig. 6 beschrieben. Zuerst wird das Linker-Phosphoramidit-Reagenz (z.B. 2-[2-(4,4'-dimethoxytrityloxy)ethylsulfonyl]ethyl-(2-cyanoethyl)-(N,N-diisopropyl)-phosphoramidit)anden Oligomerarray kondensiert. Nach Oxidation, um eine stabile Phosphat-Bindung zu erzeugen (und event. Capping, um unkontrollierte Kettenverlängerungen zu verhindern) und Entfernen der eingesetzten terminalen Schutzgruppe (z.B. der Dimethoxytrityl-Gruppe durch Säure-Behandlung) wird eine Reportergruppe (z.B. eine mit Phosphoramidit derivatisierte Cy5-Gruppe = Cy5-Phosphoramidit; Fa. Pharmacia) an die spaltbare Linker-Einheit ankondensiert. Nach Oxidation, um eine stabile Phosphodiesterbindung zu bilden, wird der Array "gescannt" (z.B durch Fluoreszenz-Scanning), um die vorher angebrachte Reportergruppe nachzuweisen. Durch Abspaltung des Linkers (z.B. durch Basenbehandlung im Fall des Sulfonylethyl-Linkers) wird die Reportergruppe im gleichen Schritt mitentfernt. Vorzugsweise, aber nicht notwendigerweise, können die Phosphatschutzgruppen im gleichen Schritt oder erst später entfernt werden. Zurückgelassen wird eine Phosphateinheit als Anhängsel an einem Ende des jeweiligen Oligomerstrangs. Dann kann der Array in Standardexperimenten (z.B. in Hybridisierungen) eingesetzt werden.

Das Verfahren kann auf beliebige Oligomerraster aus Oligonukleotiden/Nucleinsäurebiopolymeren angewendet werden, z.B aus DNA, RNA und/oder Analogen davon. Unter Analogen sind beispielsweise Phosphorthioate, PNA, modifizierte DNA- oder RNA-Nucleinsäuren (z.B. 2'-O-Methyl- ) oder auch andere Nucleinsäure-Typen mit verändertem "Backbone" oder Zucker (z.B. pRNA, homo-DNA, alphapara-NA) oder deren Chimären zu verstehen. Desweiteren läßt sich das Reportergruppen-markierte Phosphoramidit auch mit dem N-terminalen Ende von Peptiden/Proteinen umsetzen und wie vorstehend beschrieben verwenden.

Die Qualitätskontrolle erfolgt beispielsweise bei einer fluoreszenten ReporterGruppe dadurch, daß der feste Träger, auf dem sich das markierte OligomerRaster befindet, mit einem für diesen Farbstoff geeigneten Lesegerät (z.B. Fluoreszenz-Mikroskop, Fluoreszenz-Scanner) ausgewertet wird. Hierbei wird vom Lesegerät für alle Oligomer-Positionen ein relativer Intensitätswert, der der relativen Menge an synthetisiertem Oligomer an dieser Stelle entspricht, ermittelt. Ein Vergleich der ermittelten relativen Intensitäten zueinander erlaubt einen quantitativen Vergleich der jeweiligen Belegung mit Oligomer an allen Stellen des Trägers bzw. gibt Auskunft über die Qualität des synthetisierten Oligomer-Rasters. Dieser Vergleich der relativen Intensitäten an den verschiedenen Positionen des Rasters erlaubt im Folgeschritt (Auswertung eines Hybridisierungs-Experiments des Oligomer-Chips mit Sonden) einen sicheren Vergleich der Intensitäten, die sich hierbei ergeben. Beispielsweise, wenn beim Qualitäts-Check für eine bestimmte Position nur eine geringe Oligomer-Konzentration relativ zu einer anderen gemessen wird, kann bei Verwendung dieses Chips in einem Hybridisierungsexperiment für diese Position erwartungsgemäß auch nur eine geringe Intensität realtiv zu anderen ermittelt werden.

Nach erfolgter Qualitätskontrolle wird die Reportergruppe wieder abgespalten. Dies erfolgt bei Reportergruppen, die über ein Sulfonylethyl-ähnliches System an die Phosphor/Phosphatgruppen gebunden sind, durch Behandlung mit einer starken Base, z.B. DBU, Ammoniak, DBN, Diisopropylethylamin.

Das Verfahren läßt sich automatisieren und ist daher zum Massenscreening großer Chipchargen geeignet.

Die Erfindung wird weiter anhand der Figuren erläutert, welche zeigen:
- Fig. 1:: Herstellung Phosphoramidit (2-Cyanoethyl-2-dansylethyl-N,N-diisopropyl)phosphoramidit)
- Fig. 2:: Kopplung des Phosphoramidits an das Oligomer
- Fig. 3:: Durchführung der Qualitätskontrolle
- Fig. 4:: Abspaltung der Reportergruppe
- Fig. 5:: Schema des erfindungsgemäßen Verfahrens (1-Schritt-Verfahren)
- Fig. 6:: Schema des erfindungsgemäßen Verfahrens (2-Schritt-Verfahren)

Die Erfindung wird anhand der folgenden Beispiele näher erläutert.

### Beispiel 1

### Qualitätskontrolle an einem Oligomerchip

### a) Herstellung von (2-Cyanoethyl-2-dansylethyl-N,N-diisopropyl)-phosphoramidit

4,83 g 2-Dansylethanol (17,3 mmol) und 5,92 ml Diisopropylethylamin (34,6 mmol) werden in 30 ml wasserfreiem Dichlormethan aufgelöst und in einem Eisbad gekühlt. Dann werden langsam 4,5 g Chlor-(2-Cyanoethoxy)-diisopropylaminophosphan (19,0 mmol) zugesetzt. Das Eisbad wurde entfernt. Es wurde weitergerührt, bis die die DC eine vollständigen Umsetzung anzeigte. Das Reaktionsgemisch wurde mit einer gesättigten Natriumbicarbonatlösung und Dichlormehtan extrahiert, die organische Phase wurde über Natriumsulfat getrocknet und eingedampft. Das so erhaltene Öl wurde mittels Flash-Chromatographie (50g SiO₂; Toluol/Ethylacetat; 0-20% Ethylacetat) gereinigt. 5,7g des Produkts wurden als fluoreszierendes Öl erhalten. Die Ausbeute betrug 69%. Das Reaktionsschema ist in Fig. 1 gezeigt.

### b) Kopplungdes(2-Cyanoethyl-2-dansylethyl-N,N-diisopropyl)-phosphoramidits an das Oligomere

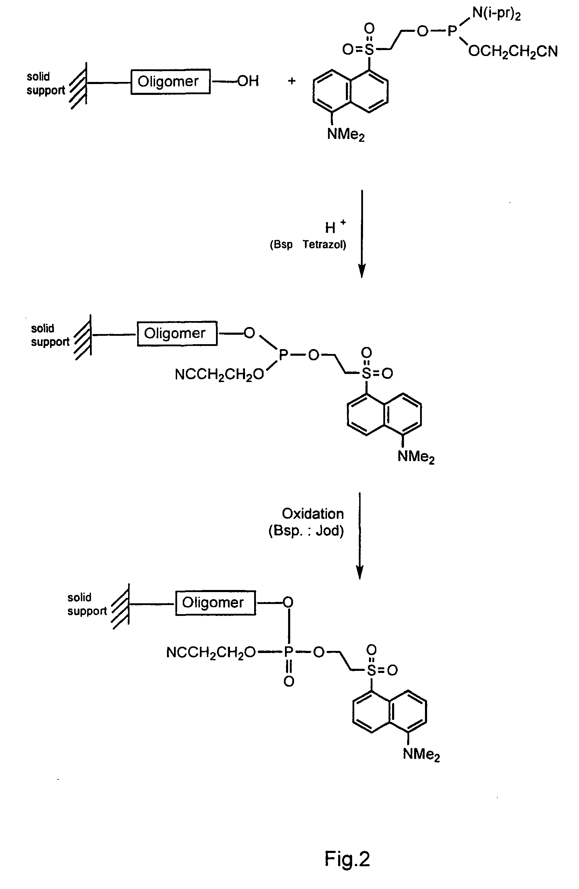

Die Ankopplung erfolgt automatisch mit Hilfe eines DNA-Synthesizers. Hierzu wird das fluoreszent-markierte Phosphoramidit analog eines 'normalen' Nucleosidbausteins (-Phosphoramidits) in trockenem Acetonitril gelöst (0.5 molar) und auf die bereits bestehenden, an der festen Phase befindliche Oligomere aufkondensiert. Dies erfolgt unter Zuhilfenahme von Tetrazol. Üblicherweise erfolgt nach der Kondensation am DNA-Synthesizer ein sog. Capping-Schritt (z.B. Reagenz Essigsäureanhydrid mit Acylierungskatalysator N-Methylimidazol), um das unkontrollierte Weiter-Wachsen der Oligomerenkette zu verhindern; dieser Schritt ist hier nicht notwendig, stört aber auch nicht, da mit dem fluoreszent-markierten Phosphoramidit ohnehin die Oligomerenkette das Ende des Wachstums erreicht hat. Wichtig ist der nachfolgende Oxidationsschritt, der die instabile Phosphor(III)- in eine stabile Phosphor(V)-Verbindung überführt. Hierzu wird eine Jod/Pyridin/THF/-Wasser-Mischung benutzt. Nun steht der so fluoreszenz-markierte DNA-Chip sofort zur qualitativen Überprüfung durch einen geeigneten Array-Reader zur Verfügung. Das Reaktionsschema ist in Fig. 2 gezeigt.

### c) Durchführung der Qualitätskontrolle

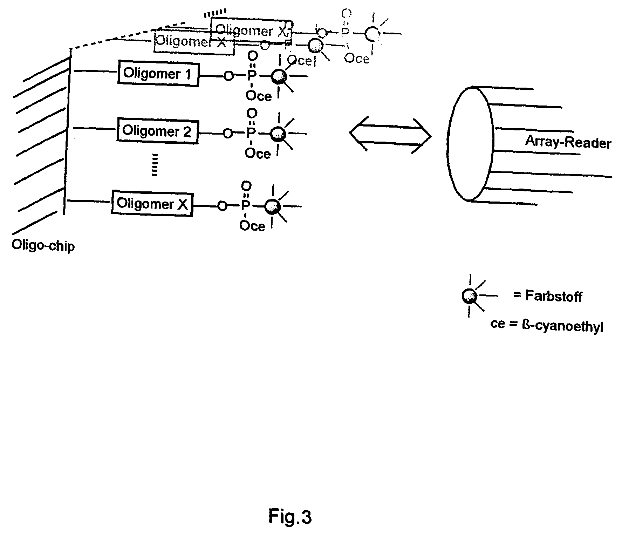

Die Qualitätskontrolle erfolgt direkt im Anschluss an (b). Hierbei wird durch Bestrahlung des Chip mit der für den Farbstoff geeigneten Wellenlänge (Dansyl: 350 nm) die Fluoreszenz angeregt. Die Emission der Fluoreszenz (Dansyl: 510 nm) wird dann durch einen geeigneten Reader (Fluoreszenzmikroskop oder Fluoreszenzscanner) für jede Position des Chip ausgelesen und quantifiziert (typischerweise Graustufenwerte). Ist z.B. an einer bestimmten Rasterposition gar keine Fluoreszenz detektierbar, obwohl dort eine Oligomerensequenz aufgebaut sein sollte, so kann daraus gefolgert werden, daß an dieser Position die Synthese nicht erfolgreich war. Dieser Chip soll dann verworfen werden. Durch Vergleich der gemessenen Intesitäten kann auf die Menge der an dieser Position synthetisierten Oligomers geschlossen werden. Dies kann dann bei der Verwendung des Oligomer-Chips in Hybridisierungsexperimenten berücksichtigt werden. So kann dann für jeden einzelnen Chip ein Qualitätsprofil durch die realtiven Fluoreszenzintensitäten aller Rasterpostionen (z.B. im Sinne einer Excel-Tabelle) erstellt werden. Dies hilft dem Anwender entsprechend bei der Auswertung seiner Hybridisierungsexperimente. Das Reaktionsschema ist in Fig. 3 gezeigt.

### d) Abspaltung der Dansylverbindung

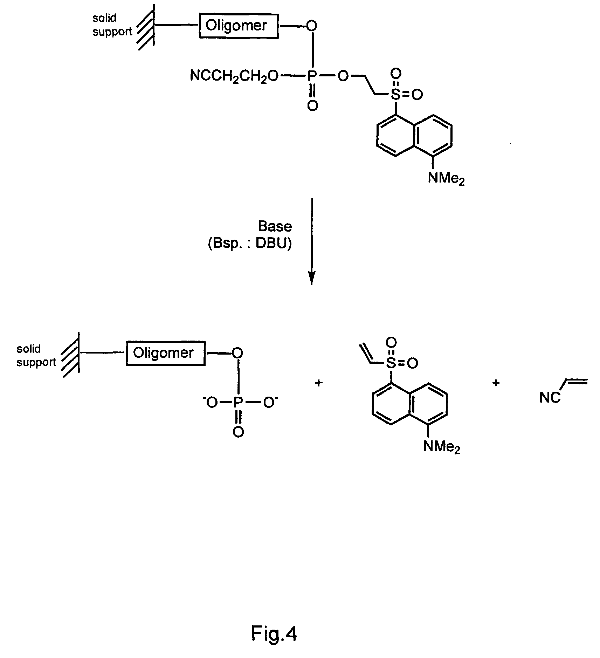

Nach erfolgtem Qualitäts-Check kann die Dansylverbindung durch Behandlung mit einer starken Base, z.B. DBU oder DBN wieder abstrahiert werden. Dies wird zweckmaessig durch Schwenken des Oligomer-Chip in der Base erreicht. Im Falle des oben angeführten Dansylfarbstoffes und unter Verwendung von 1 M DBU in Acetonitril ist das bereits spätestens nach 1-2 min erreicht. Gleichzeitig mit der Abspaltung des Dansylrestes erfolgt simultan die Abspaltung der β-CyanoethylPhosphatschutzgruppe. Die Reste (Styrol-Derivate) des Dansyl- und der Cyanoethyl-Gruppen verbleiben in der basischen Lösung und können einfach weggewaschen werden. Der DNA-Chip steht nach der Abspaltung und nach Waschen sofort für die angestrebte Verwendung (z.B. Hybridisierungsexperimente) zur Verfügung. Die am Oligomer verbleibende Phosphat-Gruppierung erhöht zudem vorteilhafterweise die Temperatur, bei der die Hybridisierungsexperimente erfolgen können. Das Reaktionsschema ist in Fig. 4 gezeigt.

## Patentansprüche

1. Verfahren zur Qualitätskontrolle von Oligomerrastern, **dadurch gekennzeichnet, daß** man an an bestimmten Rasterpositionen gebundenen Oligonukleotiden eine Phosphateinheit, die mit einer signalgebenden Reportergruppe spaltbar verknüpft ist bzw. wird, ankondensiert, anhand des Signals der Reportergruppe den Grad der Oligomersynthese bestimmt und anschließend die Reportergruppe wieder abspaltet.

2. Verfahren nach Anspruch 1, **dadurch gekennzeichnet, daß** die Oligonukleotide aus DNA, RNA und/oder Analoga bestehen.

3. Verfahren nach einem der Ansprüche 1 oder 2, **dadurch gekennzeichnet, daß** die Reportergruppe eine fluoreszierende, farbgebende, radioaktive, oder chemolumineszierende Gruppe ist.

4. Verfahren nach einem der Ansprüche 1 bis 3, **dadurch gekennzeichnet, daß** die Abspaltung der Reportergruppe unter basischen Bedingungen erfolgt.

5. Verfahren nach einem der Ansprüche 1 bis 4, **dadurch gekennzeichnet, daß** im Falle der Verwendung einer fluoreszierenden Gruppe das Signal der Reportergruppe mittels Fluoreszenz-Mikroskop oder Fluoreszenz-Scanner bestimmt wird.

6. Verfahren nach einem der Ansprüche 1 bis 5, wobei die Verknüpfung zwischen Phosphateinheit und Reportergruppe mittels eines Linkers erfolgt.

7. Abspaltbare Markierung, geeignet zur Qualitätskontrolle von Oligomerrastern, **dadurch gekennzeichnet, daß** es aus einer Phosphateinheit und einer von der Phosphateinheit abspalbaren signalgebenden Reportergruppe und ggf. einem Linker besteht.

8. Markierung nach Anspruch 7, **dadurch gekennzeichnet, daß** die signalgebende Reportergruppe eine fluoreszierende Gruppe ist.

## Claims

1. A method for controlling the quality of oligomer grids, **characterized in that** a phosphate unit linked to a signal-generating reporter group is fused to oligonucleotides bound at certain grid positions, the degree of oligomer synthesis is determined by means of the signal of the reporter group and the reporter group is then split off again.

2. The method according to claim 1, **characterized in that** the oligonucleotides comprise DNA, RNA and/or analogs.

3. The method according to claim 1 or 2, **characterized in that** the reporter group is a fluorescent, coloring, radioactive, or chemoluminescent group.

4. The method according to any of claims 1 to 3,
**characterized in that** the reporter group is split off under basic conditions.

5. The method according to any of claims 1 to 4,
**characterized in that**, when a fluorescent group is used, the signal of the reporter group is determined by means of fluorescence microscopy of fluorescence scanner.

6. The method according to any of claims 1 to 5,
**characterized in that** phosphate unit and reporter group are linked by means of a linker.

7. Cleavable labeling suitable for controlling the quality of oligomer grids, **characterized in that** it comprises a phosphate unit and a signal-generating reporter group capable of being separated from the phosphate unit and optionally a linker.

8. Labeling according to claim 7, **characterized in that** the signal-generating reporter group is a fluorescent group.

## Revendications

1. Procédé de contrôle qualité de trames d'oligomères, **caractérisé en ce qu'**on condense une unité phosphate à des oligonucléotides liés à des positions déterminées de trames, unité qui est ou devient liée de manière séparable avec un groupe reporter délivrant un signal, grâce au signal du groupe reporter le degré de synthèse de l'oligomère est déterminé et finalement le groupe reporter est libéré de nouveau.

2. Procédé selon la revendication 1, **caractérisé en ce que** l'oligonucléotide est constitué par l'ADN ou l'ARN ou des analogues.

3. Procédé selon la revendication 1 ou 2, **caractérisé en ce que** le groupe reporter est un groupe fluorescent, colorant, radioactif ou chimioluminescent.

4. Procédé selon l'une des revendications 1 à 3,
**caractérisé en ce que** la séparation du groupe reporter est réalisée en milieu basique.

5. Procédé selon l'une des revendications 1 à 4,
**caractérisé en ce que** dans le cas de l'utilisation d'un groupe fluorescent le signal du groupe reporter est déterminé au microscope fluorescent ou au scanner fluorescent.

6. Procédé selon l'une des revendications 1 à 5,
**caractérisé en ce que** la liaison entre l'unité phosphate et le groupe reporter est réalisé par une combinaison.

7. Marquage séparable pour le contrôle de qualité de trames d'oligomères, **caractérisé en ce qu'**il consiste en une unité phosphate et en un groupe reporter séparable de l'unité phosphore et le cas échéant en une combinaison.

8. Marquage selon la revendication 7, **caractérisé en ce que** le groupe reporter délivrant un signal est un groupe fluorescent.
